# EUROPEAN PATENT APPLICATION

(11) **EP 2 172 230 A2**
(43) Date of publication of application: **07.04.2010**
(21) Application number: 09167492.9
(22) Date of filing: 26.07.2006
(51) Int. Cl.: A61L 27/26, A61L 27/34, A61L 29/04, A61L 29/08, A61L 31/04, A61L 31/10, A61L 31/16, A61L 29/16, A61L 27/54

(54) **Lubricious eluting polymer blend and coating made from the same**

(30) Priority: 28.07.2005 US 191868
(62) Divisional of application: 06788585.5
(71) Applicant: Cardiac Pacemakers, Inc., St. Paul, MN 55112 (US)
(72) Inventor: Bavaro, Vincent, P., Temecula, CA 92592 (US); Quiles, Nathalie, Murrieta, CA 92563 (US)
(74) Representative: Pfenning, Meinig & Partner GbR

(57) **Abstract**

The present invention relates to a drug eluting polymer blend. More particularly, the present invention relates to a medical device with a drug eluting polymer blend incorporated therein wherein the drug elution rate is controlled by cross-linking the polymer blend. Once formed, the polymer blend can be used to create a medical device by itself or in combination with other materials. The drug, or other biologically active agent, can be loaded into the blend by soaking the polymer blend in a solution containing the agent before, during or after formation of the medical device. Alternatively, the drug or agent can be loaded into one of the polymers of the polymer blend before formation of the blend or during formation of the blend. Likewise, the polymer blend can be cross-linked before or after loading the selected drug or biologically active agent.

## Description

### FIELD OF THE INVENTION

The present invention relates to a drug eluting polymer. More particularly, the present invention relates to a medical device with a drug eluting polymer blend incorporated therein wherein the drug elution rate is controlled by a combination of cross-linking the polymer blend and controlling the ratio of the components of the polymer blend.

### BACKGROUND OF THE INVENTION

It has been recognized that pills and injections may not always be the best mode of administration for drug delivery. It may sometimes be difficult with pills and injections to obtain constant drug delivery. In addition, patient noncompliance with instructions is also a problem. Other methods of drug delivery have therefore been realized, such as drug coated stents and leads and electrodes designed to deliver drugs through ports.

Patents disclosing devices and materials for directly delivering drugs in situ include, such as, for example, U.S. Pat. Neo. 5,176,907 issued to Leong and U.S. Pat. No. 4,972,848 issued to Di Domenico et al., both of which are incorporated herein by reference for all that they teach and disclose. In Leong a poly(phosphoester-urethane) composition releases the drug through biodegradation of the polymer. Other patents to Leong include U.S. Patent Nos. 5,194,581 and 5,256,765, both of which are incorporated herein by reference for all that they teach and disclose. In Di Domenico a drug is secured in a polymer matrix before it is cured to form a solid material. The drug then releases from the polymer upon insertion into the body. Another patent disclosing drug delivery devices includes U.S. Patent No. 4,711,251 to Stokes, which is incorporated by reference for all it teaches and discloses. In Stokes a steroid is delivered through a distally facing port or ports extending through the surface of the electrode.

An important consideration in using drug delivery devices of this nature may be the release rate of the selected drug or biologically active agent. It is desirable that an effective therapeutic amount of the drug be released from the coating for the desired period of time. Burst release, a high release rate immediately following insertion into the body is undesirable in many situations and can be persistent problem. A burst release may "waste" the drug by releasing an undesirable amount of the drug over a short time frame. U.S. Pat. No. 6,258,121 B1 to Yang et al., incorporated herein by reference for all it teaches and discloses, dealt with this problem by blending two polymers with differing release rates and incorporating them into a single layer. Each component, however, had to separately be compatible for absorbing the drug and later releasing the drug.

The release mechanism of the drug from the polymeric material may depend on the nature of the polymeric material and the drug to be incorporated. The drug diffuses through the polymer to the polymer-fluid interface and then into the fluid. Release can also occur through degradation of the polymeric material such that it erodes with the drug into the body.

### BRIEF SUMMARY OF THE INVENTION

The present invention includes a guidewire with a drug eluting coating including a guidewire body and a coating disposed over at least a portion of said guidewire body wherein the coating is a blend of a polyethylene oxide with a molecular weight between about 100,000 and about 8,000,000 and a polyether block amide, the blend including up to 60% by weight of the polyethylene oxide, the coating further including a biologically active agent loaded into the blend.

Another embodiment of the present invention method of forming a medical device that releases a biologically active agent over a desired period of time includes preparing a finely dispersed and substantially uniform polymer blend of polyethylene oxide, the polyethylene oxide molecular weight between about 100,000 and about 8,000,000, and a polyether block amide, the blend including up to 60% by weight of the polyethylene oxide, forming a medical device wherein at least one portion includes the polymer blend, and loading a biologically active agent into the blend.

Another embodiment of the drug eluting polymer includes a finely dispersed blend of a polyethylene oxide with a molecular weight of between about 100,000 and about 8,000,000 and a polyurethane, the blend including up to about 60% by weight of the polyethylene oxide, the blend including a biologically active agent whereby the biologically active agent is released upon exposure to a solvent.

While multiple embodiments are disclosed, still other embodiments of the present invention will become apparent to those skilled in the art from the following detailed description, which shows and describes illustrative embodiments of the invention. The present invention is capable of modifications in various obvious aspects, all without departing from the spirit and scope of the present invention. Accordingly, the drawings and detailed description are to be regarded as illustrative in nature and not restrictive.

### BRIEF DESCRIPTION OF THE DRAWINGS

FIG. 1 illustrates a scanning electron microscope digital image of a polymer blend of the present invention.

FIG. 2 illustrates a scanning electron microscope digital image a comparison polymer blend.

FIG. 3A illustrates a two-layer tube made with the present Invention polymer blend.

FIG. 3B illustrates another two-layer tube made with the present invention polymer blend.

FIG. 3C illustrates a one-layer tube made with the present invention polymer blend.

FIG. 4 illustrates the hydration rate of tubes made with one embodiment polymer blend of the present invention and cross-linked a various amount.

FIG. 5 is an illustration representing an example of how a drug is released over time by a tube formed of the drug eluting polymer.

### DETAILED DESCRIPTION OF THE INVENTION

The present invention is a formulation for and method of making a lubricious hydrophilic drug eluting polymer blend and medical devices incorporating the same. The lubricious drug eluting hydrophilic polymer blend may be referred to as a "polyblend," a "hydrophilic drug eluting polyblend," or a "lubricious drug eluting polymer." In addition, the polyblend may be referred to as a hydrogel after cross-linking and exposure to a suitable aqueous solvent. A hydrogel is a colloidal gel in which the particles are dispersed in an aqueous solvent but where the gel only loses a little or none of its structure to the solution.

The polyblend includes a combination of a lubricious water soluble polymer that has drug absorbing and drug eluting characteristics and an insoluble polymer. The two polymers are melt mixed and solidified to form a finely dispersed polyblend. The present invention polyblend can be utilized to provide a coating on a medical device formed by extruding, co-extruding, injection molding, or die forming, or, in further embodiments, the polyblend can be directly coated on a medical device by dip coating. Embodiments of the coating formed using the polyblend of the present invention may be more robust and allow for superior performance compared to previously taught coatings. As may be appreciated, the lubricious hydrophilic drug eluting polyblend may be formed with or on any type of underlying structural article or framework.

Medical devices can include articles, such as, for example guidewires, catheters, catheter tips, sheaths, tubes, lead jackets and rings, molded articles, etc. that incorporate the present invention polyblend may help to reduce damage to the body during insertion because the lubricious nature of the drug eluting polyblend will exert reduced frictional forces. In addition, the drug eluting properties of the coating provide a site specific apparatus and method for delivery of drugs and other biologically active agents. Such medical devices may also help to reduce blood clotting as well. The polyblend is not released or abraded away during use because the lubricious drug eluting polymer is captured in the structural (or matrix) polymer.

The selection of the lubricious drug eluting water-soluble polymer may depend on a number of factors. The lubricious drug eluting polymer is partially miscible in the structural polymer but not completely miscible. When the lubricious polymer is only partially miscible rather than completely miscible the final polyblend will retain pockets of the water soluble material dispersed throughout the polyblend. The water soluble polymer may also have a lower melting point and therefore a lower viscosity at a given temperature than the structural polymer. The lower viscosity water soluble polymer is more likely to migrate towards the outer surface of the polyblend. In addition, a water soluble hydrophilic material may be able to absorb many times its own weight in water.

The molecular weight may affect the lubricity and or drug absorbing and elution properties of the final compound and so may be a factor in polymer selection. Extrusion grade resins may be of a higher molecular weight and therefore have more melt strength and will have more easily processed melt flow properties.

A lubricious hydrophilic drug eluting polymer includes polyethylene oxide (PEO). Other lubricious materials may also be incorporated, such as polypropylene oxide (PPO), poly ethyl oxazilone, polyethylvinylalcohol (EVOH), polyethylvinylacetate (EVA), polyvinylpyrolidone (PVP), cellulosic polymers, and other water-soluble lubricious polymers known to those skilled in the art may also be incorporated.

Structural polymers may include polyamides, polyurethanes, polyesters, olefin derived copolymers, polyethylene, high-density polyethylene (HDPE), natural and synthetic rubbers, styrenics, thermoplastic elastomers, and other specialty polymers. Polyamides may include homopolymers and copolymers like Nylon® 12 and 11, Pebax®, and Vestamid® resins. Nylon® 11 and Nylon® 12 copolymers may range in shore hardness from about 80D to 25D. Pebax is a polyether block amide manufactured by Arkema, Philadelphia, Pa. and is available in a variety of durometers. Polyurethanes may include polyesterurethanes and polyetherurethanes, like Pellethane® or Texin. One structural polymer may include polyetherurethane with a shore hardness from about 75D to 90A. Polyesters may include polyethylene terephthalate, polybutylene terephthalate, and co-polyesters like Hytrel® and Arnitel®. Rubbers may include silicone or Santoprene®. Thermoplastic elastomers may include commercially available materials like Kraton®.

The ratio of the different polymers in the polyblend helps to control the swell and hydration rates. Furthermore, cross-linking the polyblend a predetermined amount controls the hydration rates and the swell of the polyblend. The cross-linking may be between the PEO and itself, between the Pebax and itself and/or the PEO grafting to the Pebax. In further embodiments stabilizers may be included in the polymer blend, such as, for example, Irganox B225 or 1098. The polymer blend may also be formulated to include other advantageous materials, such as additional stabilizers, drugs, mixing aids, flow aids, plasticizers, heat stabilizers, antimicrobial agents, etc. In further embodiments, other anti-oxldants or other types of additives may also be utilized.

One polyblend of the present invention may include about 30 to about 60% PEO by weight. The polyblend may furthermore contain 35-50%, 40-50% PEO, or, particularly, about 40% PEO. The PEO is preferably greater than 100,000 MW. The PEO may include a molecular weight of about 200,000 to about 8,000,000, more particularly about 500,000 to 2,000,000, or, more particularly, about 1,000,000. In still further embodiments, the polyblend may be diluted during the extrusion or other medical device forming process to form materials with a lower weight percent of the hydrophilic polymer.

**EXAMPLES**

Example 1

A PEO with a molecular weight of 7,000,000(Dow WSR 303) was selected as the hydrophilic polymer and Pebax 72D was selected for the structural polymer. The final hydrophilic polymer blend included PEO at 40% by weight.

| **Step** | **Setting** |
|---|---|
| **Resin Drying Process** | |
| Drying Temp | 170°F (Pebax)/60°C (PEO) |
| Drying Time | 4 hrs. |

| **Feeder Parameters** | |
|---|---|
| PEO (7 million MW) | 70 grams/minute |
| Pebax 72D | 93 grams/minute |

| **Extruder Parameters** | |
|---|---|
| Zone #1 | 320°F |
| Zone #2 | 355°F |
| Zone #3 | 375°F |
| Zone #4 | 375°F |
| Die Zone (#5) | 375°F |
| **Screw Speed** | 200 rpm |

| **Extruder Responses** | |
|---|---|
| Drive Torque | 72% |
| Extruder Output | 22 lbs./hr |
| Die Pressure | 350 psi |
| **Melt Temp.** | 373°F |

| **Process Water Temps** | |
|---|---|
| Roll Temp | 45°F |
| Chiller Temp | 45°F |

| **Pelletizing Process** | |
|---|---|
| Pelletizer Speed | 210 rpm |

The polyether block amide was first dried at 170 °F for four hours. The PEO was dried at 60 °C in a vacuum oven (<25 mbar) for four hours. The drying time in the present example and all of the examples below can be for about the listed time or longer. The polymer materials were then separately loaded into two feeders controlled by a feeder control for addition to the compounding extruder. The compounding extruder was a Werner and Pfliedere ZSK30 co-rotating twin screw extruder. The extruder was equipped with a low shear/low energy screw that included two mixing zones, one dispersive and one distributive, each with six elements. The aspect ratio of the selected screw was 30:1 length:diameter and included modular conveying and mixing elements.

The PEO feeder was set at 70 grams/minute and the Pebax feeder was set at 93 grams/minute. The mixing barrel included four heat zones. The various heat zones and the screw type and rate allowed the material to be mixed and homogenized before it was passed through the die. The temperature zones of the barrel ranged from 320 to 375 °F. The extruder response had a drive torque of 72% and the extruder output was 22 pounds per hour. The die temperature was set at 375 °F and the die pressure response at 350 psi. The die temperature zone and pressure can be controlled to insure a desired strand viscosity. The extensional viscosity (i.e., melt strength) of the material when it passed through the die was adjusted so that the produced polyblend strand maintained its shape until it was properly cooled.

In the present embodiments a die with four holes of 0.180" inch diameter was utilized to form the polyblend into four concurrent strands. The extruded polyblend strands were then drawn out and cooled on the chill roller. Each strand was drawn to 0.100" before being pelletized. A cooled water/glycol solution (1:1) chilled each roller but in the present embodiment no water touched the hydrophilic polyblend during the cooling process. The chill rolls utilized were Davis Standard laboratory grade three-roll stack sheet extrusion rollers. The pelletizer was a Gala strand pelletizer.

Once the molten strands were solidified the polymer blend was chopped/cut into pellets by the pelletizer set at 210 rpm.

Example 2

A PEO with a molecular weight of 7,000,000 was utilized as the hydrophilic polymer and Pebax 72D was selected as the structural polymer. The final hydrophilic polymer blend included PEO at 40% by weight.

| **Step** | **Setting** |
|---|---|
| **Resin Drying Process** | |
| Drying Temp | PEO - 50°C with vacuum Pebax - 160°F with desiccant forced air |
| Drying Time | 12 hrs |

| **Feeder Parameters** | |
|---|---|
| PEO (7 million MW) | 50 grams/minute |
| Pebax 72D | 75 grams/minute |

| **Extruder Parameters** | |
|---|---|
| Zone #1 | 320°F |
| Zone #2 | 355°F |
| Zone #3 | 370°F |
| Zone #4 | 360°F |
| Die Zone (#5) | 360°F |
| **Screw Speed** | **152 rpm** |

| **Extruder Responses** | |
|---|---|
| Drive Torque | 79% |
| Extruder Output | 16.5 lbs./hr |
| Die Pressure | 500 psi |

| **Process Water Temps** | |
|---|---|
| Roll Temp | 32°F |
| Chiller Temp | 32°F |

| **Pelletizing Process** | |
|---|---|
| Pelletizer Speed | 160 rpm |

Example 3

The next example utilized a PEO with a molecular weight of 7,000,000 and Pebax 72D. The final hydrophilic polymer blend included PEO at 60% by weight

| **Step Setting** | |
|---|---|
| **Resin Drying Process** | |
| Drying Temp | PEO - 50°C with vacuum Pebax - 160°F with desiccant forced air |
| Drying Time | 24 hrs / 24 hrs |

| **Feeder Parameters** | |
|---|---|
| PEO (7 million MW) | 75.0 grams/minute |
| Pebax 72D | 50.0 grams/minute |

| **Extruder Parameters** | |
|---|---|
| Zone #1 | 320°F |
| Zone #2 | 355°F |
| Zone #3 | 370°F |
| Zone #4 | 360°F |
| Die Zone (#5) | 360°F |
| **Screw Speed** | 175 rpm |

| **Extruder Responses** | |
|---|---|
| Drive Torque | 81% |
| Extruder Output | 16.5 lbs./hr |
| Die Pressure | 680 psi |

| **Process Water Temps** | |
|---|---|
| Roll Temp | 32°F |
| Chiller Temp | 32°F |

| **Pelletizing Process** | |
|---|---|
| Pelletizer Speed | 130 rpm |

Example 4

A PEO with a molecular weight of 1,000,000 (Dow WSR N12K) was utilized as the hydrophilic polymer and Pebax 72D was the structural polymer. The final hydrophilic polymer blend included PEO at 40% by weight.

| **Step** | **Setting** |
|---|---|
| **Resin Drying Process** | |
| Drying Temp | PEO - 50°C with vacuum Pebax - 160°F with desiccant forced air |
| Drying Time | 12 hrs |

| **Feeder Parameters** | |
|---|---|
| PEO (1,000,000 MW) | 50 grams/minute |
| Pebax 72D | 75 grams/minute |

| **Extruder Parameters** | |
|---|---|
| Zone #1 | 320°F |
| Zone #2 | 355°F |
| Zone #3 | 370°F |
| Zone #4 | 360°F |
| Die Zone (#5) | 360°F |
| **Screw Speed** | 175 rpm |

| **Extruder Responses** | |
|---|---|
| Drive Torque | 65% |
| Extruder Output | 16.5 lbs./hr |
| Die Pressure | 420 psi |

| **Process Water Temps** | |
|---|---|
| Roll Temp | 32°F |
| Chiller Temp | 32°F |

| **Pelletizing Process** | |
|---|---|
| Pelletizer Speed | 120 rpm |

Example 5

A PEO with a molecular weight of 200,000 (Dow WSR N80) was mixed with Pebax 72D. The final hydrophilic polymer blend included PEO at 40% by weight.

| **Step** | **Setting** |
|---|---|
| **Resin Drying Process** | |
| Drying Temp | PEO - 50°C with vacuum Pebax - 160°F with desiccant forced air) |
| Drying Time | 24 hrs / 24 hrs |

| **Feeder Parameters** | |
|---|---|
| PEO (200,000 MW) | 50.0 grams/minute |
| Pebax 72D | 75.0 grams/minute |

| **Extruder Parameters** | |
|---|---|
| Zone #1 | 320°F |
| Zone #2 | 355°F |
| Zone #3 | 370°F |
| Zone #4 | 360°F |
| Die Zone (#5) | 360°F |
| **Screw Speed** | 173 rpm |

| **Extruder Responses** | |
|---|---|
| Drive Torque | 58% |
| Extruder Output | 16.5 lbs./hr |
| Die Pressure | 250 psi |
| **Melt Temp.** | 352°F |

| **Process Water Temps** | |
|---|---|
| Roll Temp | 32°F |
| Chiller Temp | 33°F |
| **Pelletizing Process** | |
| Pelletizer Speed | 125 rpm |

Example 6

The hydrophilic polymer was a PEO with a molecular weight of 1,000,000 and the structural polymer was Pebax 72D. The final hydrophilic polymer blend included PEO at 40% by weight.

| **Step** | **Setting** |
|---|---|
| **Resin Drying Process** | |
| Drying Temp | PEO - 50°C with Vacuum Pebax - 160°F with desiccant forced air |
| Drying Time | 36 hrs / 24 hrs |

| **Feeder Parameters** | |
|---|---|
| PEO (1,000,000 MW) | 50.0 grams/minute |
| Pebax 72D | 75.0 grams/minute |

| **Extruder Parameters** | |
|---|---|
| Zone #1 | 320°F |
| Zone #2 | 355°F |
| Zone #3 | 370°F |
| Zone #4 | 360°F |
| Die Zone (#5) | 360°F |
| **Screw Speed** | 170 rpm |

| **Extruder Responses** | |
|---|---|
| Drive Torque | 70% |
| Extruder Output | 16.5 lbs. /hr |
| Die Pressure | 520 psi |
| **Melt Temp.** | 341°F |

| **Process Water Temps** | |
|---|---|
| Roll Temp | 32°F |
| Chiller Temp | 33°F |

| **Pelletizing Process** | |
|---|---|
| Pelletizer Speed | 125 rpm |

Example 7

A PEO of with a molecular weight of 7,000,000 was mixed with HDPE as the structural polymer. The HDPE was a Quantum HDPE 6007 (0.6 MFI) (Philips Slurry process). The final hydrophilic polymer blend included PEO at 35% by weight. Only the PEO was dried as the HDPE is hydrophobic. The PEO was dried under a vacuum to approximately 0.03 weight percent water.

| **Step** | **Setting** |
|---|---|
| **Resin Drying Process** | |
| Drying Temp | Vacuum (PEO) |
| Drying Time | |

| **Feeder Parameters** | |
|---|---|
| PEO (7,000,000 MW) | 56 grams/minute |
| HDPE | 104 grams/minute |

| **Extruder Parameters** | |
|---|---|
| Zone #1 | 375°F |
| Zone #2 | 430°F |
| Zone #3 | 450°F |
| Zone #4 | 450°F |
| Die (#5) | 450°F |
| **Screw Speed** | 134 rpm |

| **Extruder Responses** | |
|---|---|
| Die Pressure | 490 psi |
| Torque | 45% |
| Output | 20 lbs./hr |
| **Melt Temp.** | 456°F |

| **Process Water Temps** | |
|---|---|
| Chiller Temp | 60°F |
| Fluid Temp | 50°F |

| **Pelletizing Process** | |
|---|---|
| Cutter Speed | 190 (rpm) |

Example 8

A PEO with a molecular weight of 7,000,000 was mixed with Pebax 72D. The final hydrophilic polymer blend included PEO at 35% by weight. Both materials were dried under a vacuum to approximately 0.03 weight percent water.

| **Step** | **Setting** |
|---|---|
| **Resin Drying Process** | |
| Drying Temp | Vacuum |
| Drying Time | |

| **Feeder Parameters** | |
|---|---|
| PEO (7,000,000 MW) | 84 grams/minute |
| Pebax | 157 grams/minute |

| **Extruder Parameters** | |
|---|---|
| Zone #1 | 320°F |
| Zone #2 | 335°F |
| Zone #3 | 385°F |
| Zone #4 | 360°F |
| Die (#5) | 360°F |
| **Screw Speed** | 175 rpm |

| **Extruder Responses** | |
|---|---|
| Die Pressure | 510 psi |
| Torque | 87% |
| Output | 30 lbs./hr |
| **Melt Temp.** | 360°F |

| **Process Water Temps** | |
|---|---|
| Roll Temp | 50°F |
| Chiller Temp | 74°F |

| **Pelletizing Process** | |
|---|---|
| Cutter Speed | 290 (rpm) |

Example 9

In the ninth example a PEO with a molecular weight of 7,000,000 was utilized as the hydrophilic polymer and HDPE was the structural polymer. The final hydrophilic polymer blend included PEO at 40% by weight. The PEO was dried under a vacuum to approximately 0.03 weight percent water. The produced strands were cooled by conventional means, which included running the strands through a water bath at approximately room temperature and then pelletized.

| **Step** | **Setting** |
|---|---|
| **Resin Drying Process** | |
| Drying Temp | Vacuum |
| Drying Time | |

| **Feeder Parameters** | |
|---|---|
| PEO (7,000,000 MW) | 7.4 grams/minute |
| HDPE | 4.8 grams/minute |

| **Extruder Parameters** | |
|---|---|
| Zone #1 | 375°F |
| Zone #2 | 422°F |
| Zone #3 | 431°F |
| Zone #4 | 401°F |
| Die (#5) | 400°F |
| **Screw Speed** | 175 rpm |

| **Extruder Responses** | |
|---|---|
| Die Pressure | 530 psi |
| Torque (%) | 80% |
| Output (#/hr) | 12 lbs./hr |
| **Water Bath Temp** | Room Temp Bath |

Example 10

A PEO with a molecular weight of 7,000,000 was utilized as the hydrophilic polymer and Pebax 72D was the structural polymer. The final hydrophilic polymer blend included PEO at 40% by weight. The produced strands were cooled by running on a conveyor belt cooled with an air conditioner and then pelletized.

| **Step** | **Setting** |
|---|---|
| **Resin Drying Process** | |
| Drying Temp | 200° for PebaxNacuum for PEO |
| Drying Time | 12 hrs / 12 hrs |

| **Feeder Parameters** | |
|---|---|
| PEO | 6 grams/minute |
| Pebax | 4.0 grams/minute |

| **Extruder Parameters** | |
|---|---|
| Zone #1 | 350°F |
| Zone #2 | 400°F |
| Zone #3 | 400°F |
| Zone #4 | 380°F |
| Die (#5) | 380°F |

| **Conveyance Settings** | |
|---|---|
| Screw Speed | 200 |

| **Extruder Responses** | |
|---|---|
| Die Pressure | 390 psi |
| Torque | 90% |
| Output | 9.99 lbs./hr |
| **Belt Temp** | AC cooling - forced air |

Example 11

A hydrophilic polyblend was formed utilizing 20% by weight PEO, with a molecular weight of 7,000,000, with 80% by weight Pellethane 90A. The two polymers were mixed with about 2% by weight triallyl triazine trione (Aldrich Chemical Co., Milwaukee, WI) and about 0.2% by weight Irganox 1098. The triallyl triazine trione is a cross linking promoter.

In the present embodiment the materials were mixed by tumbling the Pellethane 90A and the triallyl triazine trione for 15 minutes, adding the Irganox 1098 and tumbling for and additional 15 minutes, and then adding the PEO and tumbling for an additional 30 minutes. The mixed materials were then all added to the extruder through one feeder.

| **Step** | **Setting** |
|---|---|
| **Resin Drying Process** | |
| Drying Temp | PEO - 78°F with vacuum Pellethane 150°F with forced air desiccant |

| **Feeder Parameters** | |
|---|---|
| All materials | 60 grams/minute |

| **Extruder Parameters** | |
|---|---|
| Zone #1 | 335°F |
| Zone #2 | 355°F |
| Zone #3 | 365°F |
| Zone #4 | 365°F |
| Die (#5) | 380°F |

| **Conveyance Setting** | |
|---|---|
| Screw Speed | 94 rpm |

| **Extruder Responses** | |
|---|---|
| Die Pressure | 141 psi |
| Torque | 15.8% |
| **Process Temps** | Belt cooled with A/C |

| **Pelletizing Process** | |
|---|---|
| Cutter Speed | 167 (rpm) |

### Comparison Example

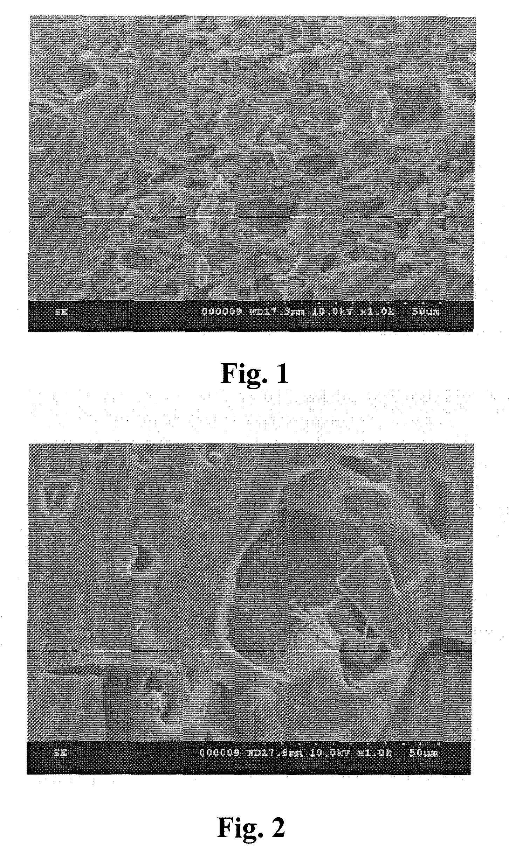

The miscibility characteristics of PEO in Pebax versus PVP in Pebax were compared. A polyblend of 40% PEO (WSRN12K, 1,000,000 MW)/60% Pebax 72D with 0.2 pphr (parts per hundred resin) Irganox B225 was formulated to compare with a polyblend of 40% PVP (K-90, 900,000 - 1,700,000 MW)/60% Pebax 72D with 0.2 pphr Irganox B225. After creation of each polyblend the material was extruded into a cylinder and cross-sectioned. The samples were placed in water at room temperature for 8 hours to dissolve the hydrophilic phase. Each sample was then dehydrated for eight hours in a vacuum oven and gold coated for viewing under a scanning electron microscope. FIG. 1 illustrates the SEM of the PEO polyblends. The PEO showed consistent and uniform dispersion in the structural material. As shown, the PEO and the Pebax form a finely dispersed blend that is substantially uniform. FIG. 2 illustrates the PVP polyblend. From the image of the PVP polyblend it is apparent that the PVP droplets are not of a uniform size, are larger, and are poorly dispersed. Clumps of the PVP material were further visible to the unaided eye. The PEO therefore creates a more finely dispersed polyblend material. The PVP polyblend is therefore less miscible in the polyether block amide structural polymer and presents a less uniformly lubricious surface. In addition, large irregular deposits of PVP on the surface make the polyblend brittle and thus easier to break off during use and may be possibly released as a contaminant into the body.

Each of the co-extruded PEO and PVP polyblends were also tested for suitability in a medical device. The PVP polyblend displayed brittle characteristics and could only be elongated less than 5%. The brittleness of the compound rendered it unacceptable for medical device applications. Moreover, upon hydration in 37°C water the co-extruded PVP became only moderately lubricious to the hand.

In comparison, the PEO polyblend rendered a tough compound with high strength. The PEO polyblend was tested and showed elongation values greater than 80%. The tough properties of the PEO polyblend also made it useful for medical device applications. Moreover, upon hydration in 37°C water the PEO polyblend became very lubricious to the hand. PEO polyblends, therefore, are superior to PVP polyblends for medical device lubricating applications.

### Medical Devices Formed From the Polyblend

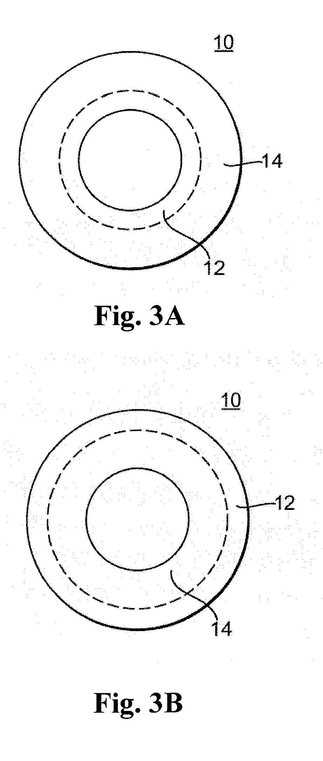

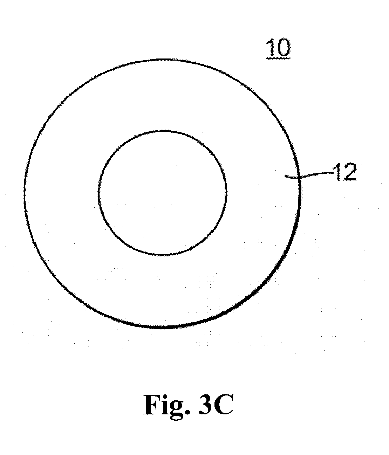

FIG. 3A illustrates a dual layer tube 10 with a polyblend layer 12 as the inner layer formed from the PEO polyblend and a polymer layer 14 as the outer layer formed of a structural polymer. The tube 10 may be formed by co-extrusion and the polyblend layer 12 may be between about 0.001-0025 inches thick. Furthermore, the tube 10 may be incorporated into any medical device, such as, for example, a catheter, a sheath, a stent or lead delivery device, an introducer, or a dilator. The tube 10 may further be made from any of the PEO polyblend materials previously discussed. As illustrated in FIG. 3B, the polyblend layer 12 may be the outer layer. In further embodiments, two or more different ratio polyblends may be utilized to make two or more of the layers of the tube 10. As illustrated in FIG. 3C the entire tube 10 may also be entirely composed of the polyblend layer 12.

Cross-linking the polyblend layer 12 improves the retention rate of the PEO when a stent or other device is passed through the lumen of the catheter. Such cross-linking may also improve the retention rate of the lubricious surface when exposed to a solvent.

The cross-linking of the PEO polyblend may form a cross-linked polymer matrix (an interpenetrating cross-linked network) that is water swellable. Such a material may form a hydrogel when hydrated. During hydration the hydrogel will not dissolve in the aqueous solution but will become water swollen and lubricious. Determining the right amount of cross-linking energy to expose the PEO polyblend to may be determined by minimizing the amount of free PEO that dissolves during hydration. This amount may be determined by the sol point, cross link density, or percent weight loss of the hydrogel after cross-linking, hydration, and drying. As may be appreciated, various structural polymer materials may be more or less susceptible to cross-linking in this manner and may form a graft with the water soluble polymer. In addition, other agents may be added to the polyblend to improve the cross-linking and to affect the resultant structure. In additional embodiments it may be desired that the PEO be released from the polyblend at a controlled rate.

**Drug Elution From Polyblend**

The polyblends described above can be utilized as part of a medical device whereby the polyblend is impregnated (or loaded) with a drug to deliver the drug in a site specific manner. Such a medical device may include a catheter, a guidewire, catheters, catheter tips, sheaths, tubes, lead jackets and rings, molded articles, or any other medical device that is inserted into the body. When that portion of the medical device that includes the drug impregnated polymer is exposed to the body's fluids the resulting hydration of the polymer releases the drug into the body.

Coating a guidewire with the polyblend, for example, allows drug elution from a medical device that can be easily inserted into the vasculature and inserted further than many other medical devices. Other medical devices, such as tubes, may include the drug on any desired portion of its length. Moreover, the release rate of the drug from the medical device containing the polyblend can be controlled by selective cross-linking of the polyblend itself. Selectively cross-linking the polyblend controls the hydration rate and therefore the elution rate of the selected drug. The ratio of the water soluble fraction in the polyblends also effects the hydration and swell rates. Moreover, including polyblends of different ratios in the same medical device may allow for controlling the rate of elution of the selected drug.

The polyblend may be melted and utilized to directly coat a medical device using a process such as is disclosed in U.S. Pat. No. 6,695,915, which is incorporated by reference for all that it teaches and discloses. The polyblend may also be coated on the medical device by other methods known to those in the art, such as dipping the medical device directly into a melt pool of the polyblend. Various characteristics of the medical device may influence adhesion on the medical device. In further embodiments, pre-coatings or other pre-treatments may be applied to the medical device before coating with the polyblend. Moreover, cross-linking the material coated on the,medical device may reduce swelling and improve retention of the lubricious material.

The material eluted from the polyblend can be a drug or any other desired material, for example, a biologically active agent or a therapeutic agent that is compatible with the polyblend. Such agents may include steroids, antithrombotic compounds, anticoagulants, aspirin, platelet inhibitors, cholesterol lowering gents, anti-inflammatory agents, anti-allergic agents, anti-rejection agents, anti-cancer drugs, and antibodies, among others.

In one example, a 20% PEO-80% polyetherurethane (Pellethane® 90AE) polyblend material was produced as in Example 11. Once the material was formed it was extruded in the shape of a tube with an outer diameter of 0.067 inches and an inner diameter of 0.017 inches. Two pieces of the tube were cross-linked and one was not cross-linked. One piece of tube was electron beam cross-linked at 5 Mrad and another was cross linked at 10 Mrad. In one embodiment an electron beam may operate at an energy of 10⁶ EV. Such an electron beam may be applied once, twice, or more than twice to achieve the desired exposure and cross-link density. The cross-linking may also be performed by other methods know to those in the art. In the present example the triallyl triazine trione helps to promote and activate the cross linking. The degree of cross-linking may be altered in various embodiments from being highly cross-linked to not being cross-linked at all, depending on the rate of the drug elution desired and the characteristics of the drug.

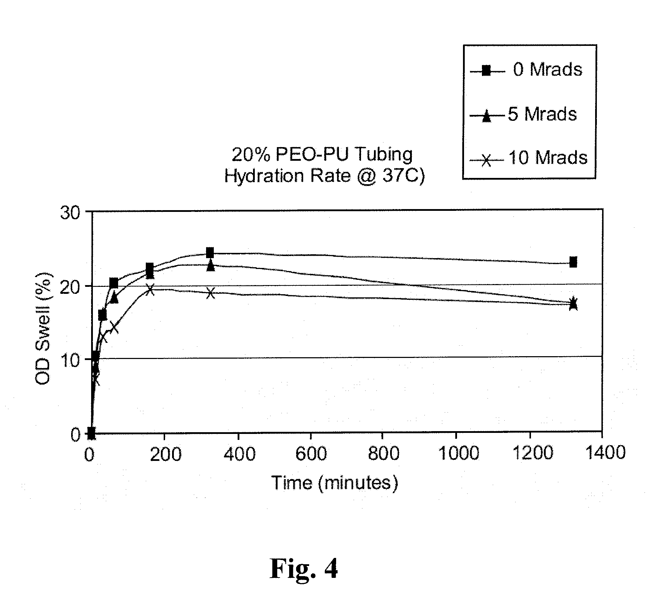

Each of the tubes were then dipped in water and measured periodically to determine their swell rate. The swell rate correlates to the hydration rate. As illustrated in FIG. 4, cross-linking the polyblend to form a matrix served to reduce the overall swell rate of the tube and therefore reduce the hydration rate. After impregnation with the desired biologically active agent, therefore, a tube that is more cross-linked will hydrate more slowly and therefore release the impregnated agent more slowly. As may be appreciated, impregnating the more cross-linked tube with the agent may be affected if the tube is cross-linked before impregnation. In certain situations the e-beam may cause certain changes in the chemical structure of the selected agent and so the e-beam cross-linking may be accomplished and the agent added later. In other situations the agent may be added to the polymer first.

In a second example, a 20% PEO-PU (Pellethane® 90AE) polyblend material was produced as in Example 11 and diluted further to make a polyblend of 8% PEO. Once the material was formed it was extruded in the shape of a tube with an outer diameter of 0.067 inches and an inner diameter of 0.017 inches. One piece of the extruded tube was electron beam cross-linked at 5 and another piece was electron beam cross-linked at 10 Mrads. A third piece was not cross-linked. Each of the tubes were then dipped in water and measured periodically to determine their swell rate. The 8% PEO material swelled less than the 20% material and also swelled at a slower rate.

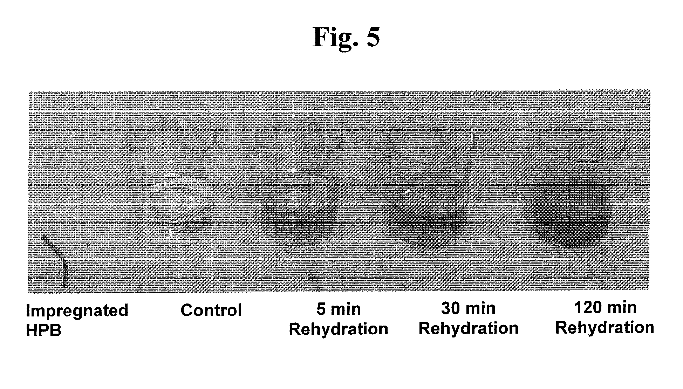

In a third example a 60% PEO/Pebax polyblend material was produced. The material was then extruded to form a tube with an outer diameter of 0.067 inches and an inner diameter of 0.017 inches. The tube was impregnated with Red Dye #4 by soaking it in a room temperature water solution for 24 hours. The tube was then dried in a vacuum oven at 60 °C for 24 hours. The impregnated polyblend tube was then cut into several equal length strips and each tube was soaked for a set amount of time at room temperature in a flask containing a water solution. FIG. 5 illustrates the flasks in which the tubes were rehydrated for the indicated times. The darker color of the solutions in which the tubes were soaked for a longer period of time indicates that more dye was released over time as the tube was hydrated.

The polyblend tube may be impregnated with the selected biologically active agent using any appropriate solvent. Because of the nature of PEO the solvent may preferably be polar. Soaking the polyblend tube for different periods of time will impregnate the tube with different amounts of the selected biologically active material up to some maximum amount. In further embodiments, the polyblend may be impregnated during melt mixing of the material, by spraying the biologically active agent directly on the tube, or by other methods. In addition, the drug or agent may be loaded into the water soluble polymer (the PEO) before or during formation of the blend if the drug or agent is able to withstand the heating and pressure required to make the blend, strand or medical device and subsequent cross-linking steps, if desired.

In further embodiments, the polyblend may be part of a tube, guidewire, or other medical device that may be telescopically extended from a catheter to deliver the selected agent at a defined location.

As may be appreciated, in further embodiments the lubricious drug eluting polyblend of the present invention may be utilized with any type of medical device known to those in the art that benefits from a drug eluting layer.

Other intravenous devices for which the present invention polyblend may impart desirable drug eluting properties may include 1) a guiding catheter shaft using the hydrophilic compound as the inner layer; 2) a polymer shunt or stent delivery device where the hydrophilic compound is the inner layer and is impregnated with an anti-coagulant agent to prevent clotting, cholesterol or other blood component build up in the arteries; 3) an implantable device (lead) outer or inner layer; and 4) a lead electrode coating.

Various modifications and additions can be made to the exemplary embodiments discussed without departing from the scope of the present invention. Accordingly, the scope of the present invention is intended to embrace all such alternatives, modifications, and variations as fall within the scope of the claims, together with all equivalents thereof. The invention relates, inter alia, to the following aspects:
1. A guidewire with a drug eluting coating comprising:
   a guidewire body; and
   a coating disposed over at least a portion of said guidewire body wherein the coating is a blend of a polyethylene oxide with a molecular weight between about 100,00 and about 8,000,000 and a polyurethane, the blend including up to 60% by weight of the polyethylene oxide, the coating further including a biologically active agent loaded into the blend.
2. The guidewire of aspect 1 wherein the blend is electron beam cross-linked before the biological agent is loaded.
3. The guidewire of aspect 1 wherein the blend includes about 40% polyethylene oxide.
4. The guidewire of aspect 1 wherein the polyethylene oxide has a molecular weight of about 1,000,000.
5. The guidewire of aspect 1 wherein the biologically active agent is a steroid.
6. The guidewire of aspect 1 further comprising a second coating disposed over at least a portion of said guidewire body wherein the coating is a blend of polyethylene oxide with a molecular weight between about 100,000 and about 8,000,000 and polyether polyurethane, the blend including up to 60% by weight of the polyethylene oxide, the coating further including a biologically active agent loaded into the blend.
7. A method of forming a medical device that releases a biologically active agent over a desired period of time comprising:
   preparing a finely dispersed and substantially uniform polymer blend of polyethylene oxide, the polyethylene oxide molecular weight between about 100,000 and about 8,000,000, and a polyurethane, the blend including up to 60% by weight of the polyethylene oxide;
   forming a medical device wherein at least one portion includes the polymer blend; and
   loading a biologically active agent into the blend.
8. The method of aspect 7 wherein preparing the polymer blend includes preparing a polymer blend using a polyethylene oxide of a molecular weight of about 200,000 to 7,000,000.
9. The method of aspect 7 wherein loading the biologically active agent further comprises:
   exposing the medical device to a solution containing the biologically active agent whereby the solution contacts the polymer blend; and
   drying the medical device such that a desired amount of the biologically active agent is retained in the polymer blend.
10. The method of aspect 7 further comprising cross-linking the polymer blend before or after loading the biologically active agent.
11. The method of aspect 7 wherein preparing the polymer blend includes preparing a polymer blend that includes about 40% polyethylene oxide.
12. The method of aspect 7 wherein preparing the polymer blend includes preparing a polymer blend using a polyethylene oxide of a molecular weight of about 1,000,000.
13. The method of aspect 7 further comprising preparing a second finely dispersed and substantially uniform polymer blend of polyethylene oxide, the polyethylene oxide molecular weight between about 100,000 and about 8,000,000, and a polyurethane, the blend including up to 60% by weight of the polyethylene oxide; and
   incorporating the second polymer blend into the medical device.
14.A drug eluting polymer comprising:
   a finely dispersed blend of a polyethylene oxide with a molecular weight of between about 100,000 and about 8,000,000 and a polyurethane, the blend including up to about 60% by weight of the polyethylene oxide, the blend including a biologically active agent whereby the biologically active agent is released upon exposure to a solvent.
15.The drug eluting polymer of aspect 14 wherein the blend is electron beam cross-linked.
16. The drug eluting polymer of aspect 14 wherein the polymer blend includes about 40% polyethylene oxide.
17.The drug eluting polymer of aspect 14 wherein the polyethylene oxide has a molecular weight of about 500,000 to 2,000,000..
18. The drug eluting polymer of aspect 14 wherein the polymer blend further includes a cross-linking agent with two or more functional sites.
19. The drug eluting polymer of aspect 14 wherein the lubricious polymer is incorporated into a medical device.
20.A drug eluting polymer comprising:
   a finely dispersed blend of a polyethylene oxide with a molecular weight of between about 100,000 and about 8,000,000 and a polyether block amide, the blend including up to about 60% by weight of the polyethylene oxide, the blend including a biologically active agent whereby the biologically active agent is released upon exposure to a solvent.

## Claims

1. A method of forming a medical device from a lubricious polymer comprising:
melt mixing a polyethylene oxide and a polyether block amide by feeding the polymers at a desired rate into a compounding extruder to form a polymer blend;
coextruding a multi-layer tubular member with the blend forming an inner layer and a structural polymer forming an outer layer;
cross-linking the polymer blend of the inner layer of the multi-layer tubular member; and
incorporating the multi-layer tubular member into the medical device.

2. The method of claim 1 further comprising adding a cross-linking agent to the blend while melt mixing the polymers.

3. The method of claim 2 wherein the cross-linking agent is a multi-functional allylic compound.

4. The method of claim 3 wherein the mutli-functional allylic compound is triallyl triazine trione.

5. The method of claim 1 wherein the polyethylene oxide and the polyether block amide are in a ratio to produce a polymer that is about 40% polyethylene oxide.

6. A method of forming a medical device that presents a lubricious surface comprising:
preparing a finely dispersed and substantially uniform polymer blend of polyethylene oxide and a water insoluble structural polymer;
coextruding a multi-layer tubular member with the blend forming an inner layer and a structural polymer forming an outer layer;
cross-linking the polymer blend of the inner layer of the multi-layer tubular member; and
incorporating the multi-layer tubular member into the medical device.

7. The method of claim 6 wherein the structural polymer is polyurethane.

8. The method of claim 6 wherein the structural polymer is polyether block amide.

9. The method of claim 6 further comprising adding a cross-linking agent to the blend while melt mixing the polymers.

10. The method of claim 6 wherein the polyethylene oxide and the polyether block amide are in a ratio to produce a polymer that is about 40% polyethylene oxide.

11. The method of any of the preceding claims wherein the polyethylene oxide has a molecular weight between about 100,000 and about 8,000,000.

12. The method of any of the preceding claims wherein the cross-linking is performed by electron beam radiation.

13. The method of claim 12 wherein the cross linking includes exposing the tubular member to between about 0.0 and about 5.0 Mrad of radiation.

14. The method of claim 12 wherein the cross linking includes exposing the tubular member to between about 0.0 and about 10.0 Mrad of radiation.

15. The method of any of the preceding claims wherein the polyethylene oxide is dried prior to the coextrusion step.
